# EUROPÄISCHE PATENTSCHRIFT

(11) **EP 1 466 584 B1**
(45) Veröffentlichungstag und Bekanntmachung des Hinweises auf die Patenterteilung: **18.06.2014**
(21) Anmeldenummer: 04007165.6
(22) Anmeldetag: 25.03.2004
(51) Int. Cl.: A61Q 5/04, A61K 8/55, A61K 8/06, A61K 8/92

(54) **Wellmittel für keratinische Fasern enthaltend Parfümöle**
Agent for waving keratinic fibers containing perfume oils
Agent pour l'ondulation des fibres kératiniques contenant des huiles de parfums

(30) Priorität: 07.04.2003 DE 10315715
(43) Veröffentlichungstag der Anmeldung: 13.10.2004
(73) Patentinhaber: Henkel AG & Co. KGaA, 40589 Düsseldorf (DE)
(72) Erfinder: Müller, Burkhard Dr., 21075 Hamburg (DE); Neubüser, Inge, 22589 Hamburg (DE)

(56) Entgegenhaltungen:
- US-A- 5 338 540
- US-A1- 2002 178 514

## Beschreibung

Die Erfindung betrifft eine Verwendung von alkoxylierten Phosphatestern zur dauerhaften Solubilisierung von Parfumölen in sulfithaltigen Wellmitteln, sowie ein entsprechendes Wellmittel, enthaltend Hydrogensulfit- und/oder sulfithaltige Verbindungen in Kombination mit mindestens einem alkoxylierten Phosphatester sowie einem Parfumöl und einem Verfahren zur Dauerverformung und gleichzeitigen Parfümierung von keratinhaltigen Fasern, insbesondere menschlichen Haaren.

Als keratinhaltige Fasern können prinzipiell alle tierischen Haare, z.B. Wolle, Roßhaar, Angorahaar, Pelze, Federn und daraus gefertigte Produkte oder Textilien eingesetzt werden. Bevorzugt wird die Erfindung jedoch im Rahmen einer dauerhaften Verformung menschlicher Haare und daraus gefertigter Perücken eingesetzt. Als Beispiele sind insbesondere die Erzeugung von Dauerwellen und die Glättung von Kraushaar zu nennen.

Die dauerhafte Verformung von keratinhaltigen Fasern wird üblicherweise derart durchgeführt, daß man die Faser mechanisch verformt und die Verformung durch geeignete Hilfsmittel festlegt. Vor und/oder nach dieser Verformung behandelt man die Faser mit einer keratinreduzierenden Zubereitung. Nach einem Spülvorgang wird die Faser dann mit einer Oxidationsmittelzubereitung behandelt, gespült und von den Verformungshilfsmitteln (Wicklern, Papilloten) befreit.

Wenn als keratinreduzierende Komponente ein Merkaptan, z.B. ein Ammoniumthioglykolat verwendet wird, spaltet dieses einen Teil der Disulfid-Brücken des Keratin-Moleküls zu -SH-Gruppen, so daß es zu einer Erweichung der Keratinfaser kommt. Bei der späteren oxidativen Fixierung werden erneut Disulfid-Brücken im Haarkeratin geknüpft, so daß das Keratingefüge in der vorgegebenen Verformung fixiert wird. Die Verwendung von Merkaptanen zur Haarverformung ist aber mit erheblichen Nachteilen verbunden.

Der hohe pH-Wert der üblichen Merkaptocarbonsäuresalz-Lösungen kann die Haarstruktur schädigen. Die Verwendung von Merkaptocarbonsäureestern kann schließlich allergische Reaktionen der Haut verursachen. Außerdem weist das Haar nach der Behandlung oft einen unangenehmen Merkaptangeruch auf, der manchmal noch nach Tagen wahrnehmbar ist. Dieser olfaktorische Nachteil der Merkaptocarbonsäuresalz-Lösungen ist durch Zusatz von Parfumölen nur leicht zu mildern, da der Eigengeruch dieser Lösungen dominant ist und sich gegenüber dem Parfumduft oftmals behauptet.

Es wurde daher schon oft vorgeschlagen, zur Haarverformung anstelle der Merkaptane Sulfit zu verwenden. Durch Hydrogensulfit-Lösungen und/oder Sulfit-Lösungen und/oder Disulfit-Lösungen werden Disulfid-Brücken des Keratins in einer Sulfitolyse nach der Gleichung 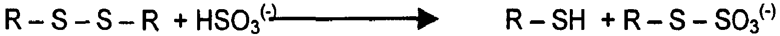 gespalten und auf diese Weise eine Erweichung der Keratinfaser erreicht. Die Spaltung kann wie zuvor geschildert in einem Fixierschritt mit Hilfe eines Oxidationsmittels unter Bildung von neuen Disulfid-Brücken wieder rückgängig gemacht werden.

Hydrogensulfit-, sulfit- bzw. disulfithaltige Wellmittel weisen nicht den starken Eigengeruch der merkaptanhaltigen Wellmittel auf. Folglich wird in den hydrogensulfithaltigen Wellmitteln eine eingearbeitete Duftnote nicht durch einen Eigengeruch des Wellmittels überlagert. Da nach der Einwirkung derartiger Wellmittel ein leichter unerwünschter Geruch im Haar zurückbleibt, besteht das Bedürfnis, derartige Wellmittel zu parfumieren. Allerdings bestehen bei der gleichmäßigen Einarbeitung von Parfümölen in sulfithaltige Wellmittel Löslichkeitsprobleme des Parfumöls. Wird ein Parfumöl zusammen mit üblichen Lösungsvermittlern in ein sulfithaltiges Wellmittel eingearbeitet, trübt sich das Wellmittel oftmals nach kurzer Zeit der Lagerung ein. Meistens ist sogar eine Auftrennung des Wellmittels in zwei diskrete Phasen mit einer Phasengrenze zu beobachten. Diese Löslichkeitsprobleme treten bei einer Einarbeitung von Parfumölen in die merkaptanhaltigen Wellmittel nicht auf.

Eine gleichmäßige Solubilisierung des Parfumöls in einem hydrogensulfit-, sulfit- bzw. disulfithaltigen Wellmittel, z.B. in gelöster Form oder als Emulsion, gewährleistet eine gleichmäßige Applikation des Parfums. Wird solch ein Wellmittel beispielsweise aus einer Vorratspackung über einen längeren Zeitraum verwendet, so bleibt das erzielte Duftergebnis über diesen Zeitraum hinweg konstant.

Die Bereitstellung eines duftenden hydrogensulfit-, sulfit- bzw. disulfithaltigen Wellmittels mit einem dauerhaft solubilisierten Parfumöl ist somit eine Herausforderung für den Fachmann. Überraschenderweise wurde gefunden, daß sich Parfumöle mit Hilfe von alkoxylierten Phosphatestern dauerhaft in derartige Wellmittel einarbeiten lassen.

In der Druckschrift WO-A1-94/24987 werden Haarbehandlungsmittel in Form einer Microemulsion, enthaltend (i) mindestens ein nichtionisches amphiphiles Tensid, (ii) ein keratinhydrolysierendes oder -reduzierendes Agens und (iii) eine Ölkomponente beansprucht. Die im Sinne dieser Lehre verwendbaren nichtionischen amphiphilen Tenside können neben einer Vielzahl anderer Tenside auch Oleth-3-Phosphat, Oleth-5-Phosphat und Oleth-10-Phosphat sein. Eine spezielle Eignung der im Rahmen der vorliegenden Erfindung ausgewählten alkoxylierten Phosphatester zur dauerhaften Solubilisierung von Parfumölen in hydrogensulfit-, sulfit- bzw. disulfithaltigen Wellmitteln wird in der oben genannten Druckschrift nicht offenbart.

Als dauerhaft solubilisiert wird ein Parfumöl im Rahmen dieser Erfindung bezeichnet, wenn es sich über einen ein Zeitraum von mindestens 90 Tagen bei Temperaturen von 0 bis +40°C in ein erfindungsgemäßes Wellmittel unter Bildung einer klaren Lösung oder Emulsion einarbeiten lässt.

Ein erster Gegenstand der Erfindung ist folglich ein keratinreduzierendes Wellmittel zur dauerhaften Verformung von keratinhaltigen Fasern, insbesondere Haaren, enthaltend
(a) bezogen auf das gesamte Mittel 5-15 Gew.-% keratin reduzierende Verbindung als Hydrogensulfit und/oder Sulfit und/oder Disulfit
(b) mindestens ein bei 20°C in reinem Wasser unlösliches Parfumöl und
(c) mindestens einen alkoxylierten Phosphatester der Formel I, 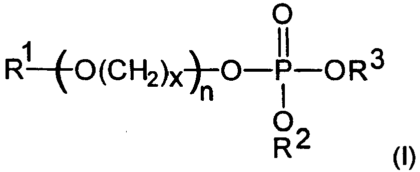 worin
   - R¹ steht für eine Arylgruppe, eine lineare oder verzweigte, gesättigte oder ungesättigte C₄-C₃₀-Fettkette,
   - R² steht für ein Wasserstoffatom, ein Alkalimetall oder 0.5 Erdalkalimetall,
   - R³ steht für ein Wasserstoffatom, eine lineare oder verzweigte, gesättigte oder ungesättigte C₄-C₃₀-Fettkette, ein Alkalimetall, 0.5 Erdalkalimetall, eine Ethylenoxid-Kette mit 6 bis 50 Ethylenoxideinheiten, eine Propylenoxid-Kette mit 6 bis 50 Propylenoxideinheiten,
   - n steht für eine ganze Zahl von 6 bis 50
   - x steht für eine Zahl 2 oder 3.

Als unlösliches Parfumöl ist erfindungsgemäß eine flüssige Parfumkomponente als Parfumöl bzw. etherisches Öl definiert, die eine Löslichkeit von weniger als 1.0 g, besonders bevorzung weniger als 0.1 g bei einer Temperatur von 20 °C in 100 g reinem Wasser besitzt.

Beispiele für die in den erfindungsgemäßen Wellmitteln enthaltenen Disulfite sind Alkalidisulfite, wie z.B. Natriumdisulfit (Na₂S₂O₅) und Kaliumdisulfit (K₂S₂O₅), Magnesiumdisulfit, Ammoniumdisulfit ((NH₄)₂S₂O₅). Ammoniumdisulfit ist ein erfindungsgemäß bevorzugtes Disulfit. Beispiele für erfindungsgemäße Hydrogensulfite sind Hydrogensulfite als Alkali-, Magnesium-, Ammonium- oder Alkanolammonium-Salz auf Basis eines C₂-C₄-Mono-, Di- oder Trialkanolamins. Ammoniumhydrogensulfit ist ein besonders bevorzugtes Hydrogensulfit. Beispiele für erfindungsgemäße Sulfite sind Sulfite als Alkali-, Ammonium- oder Alkanolammonium-Salz auf Basis eines C₂-C₄-Mono-, Di- oder Trialkanolamins. Ein bevorzugt verwendetes Sulfit ist Ammoniumsulfit. Die keratinreduzierende Verbindung wird in einer Menge von 5-15 Gew.-%, bezogen auf das gesamte erfindungsgemäße Wellmittel, eingesetzt. Der pH-Wert des Wellmittels wird auf einen Wert im Neutralbereich von pH 5 bis 8, bevorzugt von pH 6 bis 7 eingestellt. Dazu wird das schwach saure sulfithaltige Wellmittel durch eine schwache Base, z.B. ein Alkanolamin eingestellt.

Bevorzugte Alkanolamine sind erfindungsgemäß 2-Aminoethanol (Monoethanolamin) und N,N,N-Tris(2-hydroxyethyl)amin (Triethanolamin). Monoethanolamin ist ein besonders bevorzugtes Alkanolamin, das insbesondere in einer Menge von 1-6 Gew.-% bezogen auf das gesamte Wellmittel, eingesetzt wird.

In einer bevorzugten Ausführung enthält das Wellmittel zur Durchführung der Sulfitolyse 5-15 Gew.-% Ammoniumhydrogensulfit und 1-6 Gew.-% Monoethanolamin.

Bevorzugt werden diejenigen alkoxylierten Phosphatester in den erfindungsgemäßen Wellmitteln eingesetzt, in denen x gleich 2 ist.

Eine bevorzugte Arylgruppe ist die Phenylgruppe.

Besonders bevorzugte alkoxylierte Phosphatester sind Polyoxyethylenoleylether-10-EO-phosphat (INCI: Oleth-10-phosphate) mit 10 Einheiten Ethylenoxid (n=10, x=2), welcher unter dem Handelsnamen Crodafos N10 der Firma Croda vertrieben wird und Polyoxyethylenoleylether-20-EO-phosphat (INCI: Oleth-20-phosphate) mit 20 Einheiten Ethylenoxid (n=20, x=2), welcher unter dem Handelsnamen Crodafos N20 der Firma Croda vertrieben wird. Ganz besonders bevorzugt ist Polyoxyethylenoleylether-10-EO-phosphat.

Die Parfümöle sind in dem erfindungsgemäßen Mittel bevorzugt in einer Menge von 0.01 bis 4 Gew.%, besonders bevorzugt 0.1 bis 2 Gew.%, bezogen auf das gesamte Mittel enthalten. Als Beispiele für erfindungsgemäße Parfumöle sind das Parfumöl DG 13269 (BBA), DG 15453 (BBA), Natural Hair (Drom), Soft Bouquet (Drom), HAN 4859 (IFF), 98-5705 (Henkel Fragrance Center), 97-5430 (Henkel Fragrance Center), Jaune 0/298662 (Dragoco) und Aqua di Flora 0/299460 (Dragoco) zu nennen.

In einer bevorzugten Ausführungsform ist neben dem Parfumöl kein weiterer Ölkörper in dem erfindungsgemäßen Wellmittel enthalten.

Darüberhinaus kann das erfindungsgemäße Wellmittel weitere Komponenten enthalten, die die Wirkung des Sulfits auf das Keratin fördern. Solche Komponenten sind z.B. Quellmittel für keratinhaltige Fasern wie z.B. C₁-C₆-Alkohole und wasserlösliche Glykole oder Polyole wie z.B. Glycerin, 1,2-Propylenglykol oder Sorbit und Harnstoff oder Harnstoffderivate wie z.B. Allantoin und Guanidin sowie Imidazol und dessen Derivate. Bevorzugte weitere Komponenten sind 1,2-Propylenglykol, insbesondere in einer Menge von 5-20 Gew.-%, sowie Harnstoff, insbesondere in einer Menge von 1-10 Gew.-%. Die Mengenangaben beziehen sich jeweils auf das gesamte Wellmittel. In einer bevorzugten Ausführung enthält das erfindungsgemäße Wellmittel 5-20 Gew.-% 1,2-Propylenglykol und/oder 1-10 Gew.-% Harnstoff.

Weiterhin kann das erfindungsgemäße Wellmittel als Cosolubilisator mindestens einen weiteren oberflächenaktiven Stoff, der verschieden ist von den alkoxylierten Phosphatestern gemäß Formel I, aus der Gruppe der anionischen, amphoteren, zwitterionischen und nichtionischen Tenside enthalten. Diese Cosolubilisatoren haben unter anderem die Aufgabe, die Benetzung der Keratinoberfläche durch die Behandlungslösung zu fördern.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat- oder SulfonatGruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder HydroxyAlkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Fettsäuremono- und Diglyceride, an Alkylphenole mit 8 bis 15 C-Atomen in der Alkylgruppe und an Sorbitan-Fettsäureester von C₁₂-C₂₂-Fettsäuren,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Fettsäuretriglyceride, insbesondere an Rizinusöl oder gehärtetes Rizinusöl.

Weitere erfindungsgemäße nichtionische Tenside sind Alkylpolyglykoside der allgemeinen Formel R'O-(Z)ₓ. Diese Verbindungen sind durch die folgenden Parameter gekennzeichnet.

Der Alkylrest R' enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R' enthalten. Üblicherweise werden diese Verbindungen aber ausgehend von natürlichen Fetten und Ölen oder Mineralölen hergestellt. In diesem Fall liegen als Alkylreste R' Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R'
- im wesentlichen aus C₈- und C₁₀-Alkylgruppen,
- im wesentlichen aus C₁₂- und C₁₄-Alkylgruppen,
- im wesentlichen aus C₈-C₁₆-Alkylgruppen oder
- im wesentlichen aus C₁₂-C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können als Cosolubilisatoren zwitterionische Tenside verwendet werden. Als zwitterionische Tenside werden erfindungsgemäß solche oberflächenaktive Verbindungen bezeichnet, die im Molekül mindestens eine Aminogruppe und mindestens eine -COO⁽⁻⁾- oder -SO₃⁽⁻⁾-Gruppe tragen. Dabei kann die Aminogruppe erfindungsgemäß quaterniert sein. Besonders geeignete zwitterionische Tenside mit quaternierter Aminogruppe sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls geeignete zwitterionische Tenside sind solche oberflächenaktive Verbindungen, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für diese Tenside sind
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- Alkylamidoalkylamine
   mit der allgemeinen Formel C₈-C₁₈-Alkyl-CO-NH-CH₂CH₂-NR'R", worin R' steht für ein eine Gruppe -CH₂CH₂-OH oder -CH₂CH₂-O-CH₂-COOH und R" steht für eine Gruppe -CH₂-COOH, -CH₂CH₂-COOH, -CH₂CH(OH)-CH₂-SO₃H oder -CH₂CH(OH)-CH₂-O-PO₃H,
- 2-Alkylaminopropionsäuren und
- Alkylaminoessigsäuren,
mit jeweils etwa 8 bis 18 C-Atomen in der Alkylgruppe.

Besonders bevorzugte zwitterionische Tenside sind das N-Kokosfettsäureamidoethyl-N-2-hydroxyethylglycin-Natrium Salz (INCI-Bezeichnung: Disodium Cocoamphodiacetate; beispielsweise Rewoteric^{®} AM 2 C NM (Goldschmidt)), N,N-Dimethyl-N-kokosalkylammoniumbetain (INCI-Bezeichnung: Aqua (Water), Coco-Betaine); beispielsweise Dehyton® AB 30 (Cognis Deutschland), N-Kokosalkylaminopropionat, das Kokosacylaminoethylaminopropionat und das C₁₂₋₁₈-Acylsarcosin.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

In einer besonders bevorzugten Ausführung enthält das erfindungsgemäße Wellmittel als Cosolubilisator zusätzlich mindestens ein Tensid, ausgewählt aus
- Anlagerungsprodukten von 5-30 Mol Ethylenoxid und 0 bis 5 Mol Propylenoxid an lineare Fettalkohole und 1,2-Alkandiole mit 12-22 C-Atomen, an Fettsäuren mit 12-22 C-Atomen, an Fettsäuremono- und Diglyceride und an Sorbitan-Fettsäureester von C₁₂-C₂₂-Fettsäuren,
- Anlagerungsprodukten von 20-60 Mol Ethylenoxid an Fettsäuretriglyceride, insbesondere an Rizinusöl oder gehärtetes Rizinusöl,
- Betaine sowie
- Alkylamidoalkylamine
mit der allgemeinen Formel C₈-C₁₈-Alkyl-CO-NH-CH₂CH₂-NR'R", worin R' steht für ein eine Gruppe -CH₂CH₂-OH oder -CH₂CH₂-O-CH₂-COOH und R" steht für eine Gruppe -CH₂-COOH, -CH₂CH₂-COOH, -CH₂CH(OH)-CH₂-SO₃H oder - CH₂CH(OH)-CH₂-O-PO₃H.

Ganz besonders bevorzugte Cosolubilisatoren werden ausgewählt aus N-Kokosfettsäureamidoethyl-N-2-hydroxyethylglycin-Natrium Salz (INCI-Bezeichnung: Disodium Cocoamphodiacetate; beispielsweise Rewoteric® AM 2 C NM (Goldschmidt)), N,N-Dimethyl-N-kokosalkylammoniumbetain (INCI-Bezeichnung: Aqua (Water), Coco-Betaine); beispielsweise Dehyton® AB 30 (Cognis Deutschland) und Polyoxethylensorbitanmonolaurat (Tween® 20 (INCI-Bezeichnung: Polysorbate-20; Uniqema).

Darüberhinaus kann das erfindungsgemäße Wellmittel die in solchen Zubereitungen üblichen Hilfs- und Zusatzmittel enthalten. Solche Komponenten sind z.B:
- Verdickungsmittel wie z.B. Pflanzen-Gumme, Xanthan-Gum, Alginate, Cellulose- und Stärkeether,
- Proteinhydrolysate
- wasserlösliche Polymere festigender Wirkung, z.B. Polyvinylpyrrolidon, Vinylacetat/Crotonsäure-Copolymere,
- wasserlösliche kationische Polymere mit antistatischer und avivierender Wirkung, z.B. quaternierte Cellulose- oder Guarether, Dimethyldialkylammonium-Polymere, Vinylpyrrolidon-Imidazoliniummethochlorid-Copolymere, Trimethylamonioethylmethacrylat-Vinylpyrrolidon-Copolymere,
- Antischuppenwirkstoffe wie z.B. Picrotone Olamine, Zink Omadine,
- Vitamine wie z.B. Tocopherolacetat, Pyridoxin, Panthenol,
- Wirkstoffe wie Allantoin, Pyrrolidoncarbonsäuren, Pflanzenextrakte ,
- Strukturanten wie z.B. Glucose oder Maleinsäure
- pH-Einstell- und Puffermittel wie z.B. Citronensäure/Natriumcitrat, Ammoniumcarbonat, Guanidincarbonat, Phosphate,
- Komplexbildner wie EDTA, NTA, Organophosphonsäuren
- Lichtschutzmittel (UV-Absorber)
- Öl-, Fett- und Wachskomponenten oder Silikone, bevorzugt in emulgierter Form,
- Farbstoffe, Trübungs- und Perlglanzmittel sowie
- gegebenenfalls Aerosol-Treibgase

Ein zweiter Gegenstand der Erfindung ist die Verwendung von alkoxylierten Phosphatestern zur Solubilisierung von unlöslichen Parfumölen in sulfithaltigen Wellmitteln gemäß Anspruch 1. Zur Auswahl der bevorzugten alkoxylierten Phosphatester und weiterer Definitionen wird auf das zuvor Gesagte verwiesen.

Ein dritter Gegenstand der Erfindung ist ein Verfahren zur dauerhaften Verformung und gleichzeitigen Parfümierung von keratinhaltigen Fasern, insbesondere menschlichen Haaren, in dem
(i) das gegebenenfalls vorgefeuchtete Haar gegebenenfalls auf Wickelhilfen gewickelt wird,
(ii) ein Wellmittel, enthaltend
   (a) bezogen auf das gesamte Mittel 5-15 Gew.-% keratin reduzierende Verbindung als Hydrogensulfit und/oder Sulfit und/oder Disulfit
   (b) mindestens ein bei 20°C in reinem Wasser unlösliches Parfumöl und
   (c) mindestens einen alkoxylierten Phosphatester der Formel I, 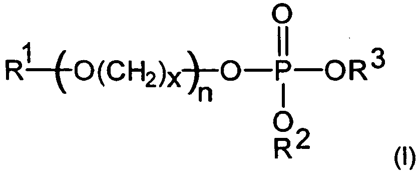 worin
      - R¹ steht für eine Arylgruppe, eine lineare oder verzweigte, gesättigte oder ungesättigte C₄-C₃₀-Fettkette,
      - R² steht für ein Wasserstoffatom, ein Alkalimetall oder 0.5 Erdalkalimetall,
      - R³ steht für ein Wasserstoffatom, eine lineare oder verzweigte, gesättigte oder ungesättigte C₄-C₃₀-Fettkette, ein Alkalimetall, 0.5 Erdalkalimetall, eine Ethylenoxid-Kette mit 6 bis 50 Ethylenoxideinheiten, eine Propylenoxid-Kette mit 6 bis 50 Propylenoxideinheiten,
      - n steht für eine ganze Zahl von 6 bis 50
      - x steht für eine Zahl 2 oder 3,
      auf das Haar aufgetragen und
(iii) nach einer Einwirkzeit vom Haar gespült wird,
(iv) das Haar gegebenenfalls mit einer oxidationsmittelhaltigen Lösung oxidativ fixiert und nach dem Entfernen der Wickelhilfen erneut gespült wird,
(v) das Haar gegebenenfalls einer Nechbehandlung mit einem Nachbehandlungsmittel unterzogen und gegebenenfalls wieder gespült wird.

Das Wellmittel, welches in dem erfindungsgemäßen Verfahren in Schritt (ii) zur Anwendung kommt, entspricht in all seinen Parametern und Ausführungsformen dem Wellmittel des ersten Gegenstandes der Erfindung.

Die Behandlung der keratinhaltigen Fasern mit dem erfindungsgemäßen Wellmittel wird bei Temperaturen von 0-60 °C über einen Zeitraum von 5-120 Minuten, bevorzugt bei Temperaturen von 20-50°C innerhalb von 10-60 Minuten durchgeführt. Zum Dauerwellen der Haare wird das Wellmittel bevorzugt auf das auf Wicklern aufgerollte Haar aufgetragen und gegebenenfalls unter Zufuhr von Wärme und unter Verwendung einer Abdeckung einwirken gelassen.

Danach werden bevorzugterweise die verformten keratinhaltigen Fasern gut mit Wasser gespült und gegebenenfalls oxidativ fixiert und erneut gespült. Dann erfolgt gegebenenfalls eine Nachbehandlung mit Wasser oder einer wäßrigen Lösung eines Puffersalzes. Im einfachsten Falle kann diese Nachbehandlung in einem längerdauernden Spülen mit Wasser mit einem pH-Wert im Bereich von 6-9 , bevorzugt von 6-8 und bevorzugt mit einer Temperatur von 20-40 °C bestehen. Die Nachbehandlung kann aber auch eine Wäsche mit einem pH-neutralen Shampoo und anschließendes Spülen mit Wasser einschließen.

Wenn es sich bei den verformten keratinhaltigen Fasern um Textilien aus Wolle oder anderen Tierhaaren handelt, kann die Nachbehandlung in der Weise erfolgen, daß man die Fasern in Wasser einweicht und gegebenenfalls leicht darin bewegt. Im Rahmen einer Dauerwellbehandlung kann man das gewickelte Haar über einen Zeitraum von wenigstens 30 Sekunden, bevorzugt mindestens 2 Minuten, mit Wasser der vorgenannten Temperatur spülen, indem man warmes Wasser über das eingerollte Haar fließen läßt. Man kann aber auch das mit Wasser oder einer wäßrigen Nachbehandlungszubereitung benetzte Haar abdecken, z.B. mit einer wasserundurchlässigen Haube oder einem Turban und gegebenenfalls unter Wärmeeinwirkung, z.B. unter einer Trockenhaube, das Wasser oder die Zubereitung auf das Haar einwirken lassen.

Wenn man für die Nachbehandlung eine geeignete kosmetische Zubereitung verwendet, die ein Puffersalz-Gemisch mit einem pH-Wert von 6 bis 9 enthält, kann man als Puffersalz z.B. Gemische von primären, sekundären und tertiären Alkaliphosphaten, Gemische aus Citronensäure und Alkalicitraten, Gemische aus Milchsäure und Alkalilactaten, Guanidinglykolat, Alkanolammoniumbenzoat und andere Salze schwacher Säuren oder schwacher Basen in einer Menge von 1-10 Gew.-% bezogen auf die wäßrige Zubereitung einsetzen.

In einer bevorzugten Ausführung der Erfindung wird vor der Nachbehandlung eine oxidative Fixierung durchgeführt. Für diesen Zweck kann man wäßrige Zubereitungen der dafür üblichen Oxidationsmittel wie z.B. Natriumbromat, Kaliumbromat oder Wasserstoffperoxid und der zur Stabilisierung wäßriger Wasserstoffperoxidzubereitungen üblichen Stabilisatoren verwenden. Der pH-Wert solcher wäßriger H₂O₂-Zubereitungen, die üblicherweise etwa 0,5 bis 3,0 Gew.-% H₂O₂ enthalten, liegt bevorzugt bei 2 bis 6. Fixiermittel auf Bromat-Basis enthalten die Bromate üblicherweise in Konzentrationen von 1 bis 10 Gew.-% und der pH-Wert der Lösungen wird auf 4 bis 7 eingestellt. Oft werden die erfindungsgemäßen Fixiermittel als Feststoffe formuliert. Sie enthalten das Oxidationsmittel dann in Form eines Festkörpers, z.B. Natriumperborat. Erst kurz vor der Anwendung werden diese Mittel dann mit Wasser versetzt. Es ist bevorzugt, als Oxidationsmittel Wasserstoffperoxid zu verwenden.

Ebenfalls möglich und bevorzugt ist, das Oxidationsmittel als 2-Komponenten-System zu formulieren. Die beiden Komponenten, von denen die eine bevorzugt eine schwach saure Wasserstoffperoxidlösung oder eine wäßrige Lösung eines anderen Oxidationsmittels ist und die andere die übrigen Bestandteile und eine Base enthält, werden ebenfalls erst kurz vor der Anwendung zu einer gebrauchsfertigen Fixierlösung mit einem pH-Wert von 7-9 vermischt (vergl. US 5,775,342).

Anstelle oder zusätzlich zu den im Zusammenhang mit dem Wellmittel genannten Tensiden können die Fixiermittel als oberflächenaktive Verbindungen auch kationische Tenside enthalten.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide, insbesondere Chloride und Bromide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Alkylamidoamine, insbesondere Fettsäureamidoamine wie das unter der Bezeichnung Tego Amid^{®} S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind die sogenannten Esterquats wie die unter dem Warenzeichen Stepantex^{®} vertriebenen Methyl-hydroxyalkyl-dialkoyloxyalkylammoniummethosulfate.

Darüberhinaus können die Fixiermittel alle bereits für die Wellmittel genannten Hilfs- und Zusatzmittel enthalten.

Sowohl Wellmittel als auch Fixiermittel können als Creme, Gel oder Flüssigkeit formuliert sein. Weiterhin ist es möglich, die Mittel in Form von Schaumaerosolen zu konfektionieren, die mit einem verflüssigten Gas wie z. B. Propan-Butan-Gemischen, Stickstoff, CO_{2,} Luft, N₂O, Dimethylether, Fluorchlorkohlenwasser stofftreibmitteln oder Gemischen davon in Aerosolbehältern mit Schaumventil abgefüllt werden.

Die folgenden Beispiele sollen den Gegenstand der Erfindung näher erläutern:

### Beispiele:

### 1. Herstellung der erfindungsgemäßen Wellmittel

Harnstoff und Citronensäure werden in Wasser unter Rühren gelöst. Anschließend werden Ammoniumbisulfit und Monoethanolamin und danach gegebenenfalls die Cosolubilisatoren, sowie 1,2-Propylenglykol und Merquat^{®} 280 unter Rühren zugegeben. Das Crodafos N10 wird in einem separaten Gefäß aufgeschmolzen und mit Monoethanolamin neutralisiert. Zu dieser Schmelze wird das Parfumöl gegeben und die Schmelze unter Zugabe von etwas Wasser geschmeidig gerührt. Anschließend wird die Schmelze unter Rühren zu der Mischung gegeben. Die Mengenangaben der einzelnen Rohstoffe sind Tabelle 1 zu entnehmen.

Es wird jeweils ein klares Wellmittel erhalten, welches sich jeweils bei einer Lagerung von 90 Tagen bei 0 bis +40 °C nicht eintrübt.

**Tabelle 1**

| **Wellmittel (erfindungsgemäß)** | **1.1 [Gew .%]** | **1.2 [Gew.%]** | **1.3 [Gew.%]** | **1.4 [Gew.%]** | **1.5 [Gew.%]** |
|---|---|---|---|---|---|
| Ammoniumbisulfit (70%-ig) | 14.30 | 14.30 | 14.30 | 14.30 | 14.30 |
| Monoethanolamin | 5.85 | 5.85 | 5.85 | 5.85 | 5.85 |
| 1,2-Propylenglykol | 5.00 | 5.00 | 5.00 | 5.00 | 5.00 |
| Harnstoff | 5.00 | 5.00 | 5.00 | 5.00 | 5.00 |
| Crodafos^{®} N10¹ | 2.00 | 1.50 | 1.00 | 1.00 | 1.00 |
| Rewoteric^{®} AM 2 C NM² | - | 0.50 | - | - | - |
| Cremophor^{®} RH40³ | - | - | 1.00 | 1.00 | - |
| Tween^{®} 20⁴ | - | - | - | 2.00 | - |
| Dehyton^{®} AB 30⁵ | - | - | - | - | 1.00 |
| Eumulgin^{®} L⁶ | - | - | 1.00 | - | 1.00 |
| Merquat^{®} 280⁷ | 0.20 | 0.20 | 0.20 | 0.20 | 0.20 |
| Citronensäure | 2.00 | 2.00 | 2.00 | 2.00 | 2.00 |
| Parfumöl⁸ | 0.60 | 0.60 | 0.60 | 0.60 | 0.60 |
| Wasser | ad 100 | ad 100 | ad 100 | ad 100 | ad 100 |

### 2. Vergleichstests

Es wurden sulfithaltige Wellmittel mit merkaptanhaltigen Wellmitteln, sowie mit sulfithaltigen Wellmitteln, enthaltend einem nicht erfindungsgemäßen alkoxylierten Phosphatester, verglichen. Dazu wurden zunächst folgende nicht erfindungsgemäße Wellmittel gemäß Tabellen 2 und 3 hergestellt.

Die Herstellung der Mittel gemäß Tabelle 2 erfolgte analog zu dem unter Punkt 1 beschriebenen Herstellverfahren.

Zur Herstellung der Mittel gemäß Tabelle 3 wurde zunächst Wasser, Ammoniumthioglykolat, Turpinal^{®} SL, Ammoniak und die entsprechenden Cosolubilisatoren (Eumulgin^{®} L, Dehyton^{®} AB, Tween^{®} 20, Lamepon^{®} S) unter Rühren gemischt und anschließend das Ammoniumhydrogencarbonat gelöst. In einem separaten Gefäß wurde das Cremophor^{®} RH 40 geschmolzen und das Parfumöl darin unter Zugabe von wenig Wasser gerührt, bis eine geschmeidige Mischung entstand. Die Schmelze wurde dann unter Rühren zu der ersten Mischung gegeben und abschließend Merquat^{®} 100 und Gluadin^{®} WQ zugefügt.

**Tabelle 2**

| **sulfithaltige Wellmittel (nicht erfindngsgrmäß)** | **2.1 [Gew.%]** | **2.2 [Gew.% ]** | **2.3 ^{a)} [Gew.% ]** | **2.4 ^{b)} [Gew.%]** | **2.5 ^{c)} [Gew.%]** |
|---|---|---|---|---|---|
| Ammoniumbisutfit (70%-ig) | 14.30 | 14.30 | 14.30 | 14.30 | 14.30 |
| Monoethanolamin | 5.85 | 5.85 | 5.85 | 5.85 | 5.85 |
| 1,2-Propylenglykol | 5.00 | 5.00 | 5.00 | 5.00 | 5.00 |
| Harnstoff | 5.00 | 5.00 | 5.00 | 5.00 | 5.00 |
| Crodafos^{®} N5A ⁹ | 2.00 | 5.00 | - | - | - |
| Eumulgin^{®} L ⁶ | - | - | 1.00 | - | 1.00 |
| Cremophor^{®} RH40 ³ | - | - | 1.00 | 1.00 | - |
| Tween^{®} 20 ⁴ | - | - | - | 2.00 | - |
| Dehyton^{®} AB 30 ⁵ | - | - | - | - | 1.00 |
| Merquat^{®} 280 ⁷ | 0.20 | 0.20 | 0.20 | 0.20 | 0.20 |
| Citronensäure | 2.00 | 2.00 | 2.00 | 2.00 | 2.00 |
| Parfumöl ⁸ | 0.60 | 0.60 | 0.60 | 0.60 | 0.60 |
| Wasser | ad 100 | ad 100 | ad 100 | ad 100 | ad 100 |

| | | | | | |
|---|---|---|---|---|---|
| zu a): wie 1.3 gemäß Tabelle 1 nur ohne Crodafos N10 zu b): wie 1.4 gemäß Tabelle 1 nur ohne Crodafos N10 zu c): wie 1.5 gemäß Tabelle 1 nur ohne Crodafos N10 | | | | | |

Alle Wellmittel gemäß Tabelle 2 waren bereits nach der Zubereitung trüb. Eine dauerhafte Solubilisierung des Parfumöls wird folglich nicht erreicht.

**Tabelle 3**

| **merkaptanhaltige Wellmittel (nicht erfindungsgemäß)** | **2.6 [Gew.%]** | **2.7 [Gew.%]** | **2.8 [Gew.%]** |
|---|---|---|---|
| Ammoniumthioglykolat (71%-ig) | 17.80 | 17.80 | 17.80 |
| Turpinal^{®} SL ¹⁰ | 0.30 | 0.30 | 0.30 |
| NH₄HCO₃ | 8.80 | 8.80 | 8.80 |
| Lamepon^{®} S¹¹ | 1.00 | 1.00 | 1.00 |
| Eumulgin^{®} L⁶ | 1.00 | 1.00 | - |
| Cremophor^{®} RH40 ³ | 1.00 | - | 1.00 |
| Tween^{®} 20 ⁴ | - | - | 1.00 |
| Dehyton^{®} AB 30 ⁵ | - | 1.00 | - |
| Gluadin^{®} WQ ¹² | 0.05 | 0.05 | 0.05 |
| Merquat^{®} 100 ¹³ | 0.05 | 0.05 | 0.05 |
| Ammoniak (25 %ige wäßrige Lösung | 1.70 | 1.70 | 1.70 |
| Parfumöl ⁸ | 0.60 | 0.60 | 0.60 |
| Wasser | ad 100 | ad 100 | ad 100 |

| | | | |
|---|---|---|---|
| ¹ Polyoxethylenoleylether-10-EO-phosphat (INCI-Bezeichnung: Oleth-10-Phosphate) (Croda) ² N-Kokosfettsäureamidoethyl-N-2-hydroxyethylglycin-Natrium Salz, ca. 39 % Aktivsubstanz (INCI-Bezeichnung: Disodium Cocoamphodiacetate) (Goldschmidt) ³ hydriertes Rizinusöl mit 40-45 EO (INCI-Bezeichnung: PEG Hydrogenated Castor Oil) (BASF) ⁴ Polyoxethylensorbitanmonolaurat (INCI-Bezeichnung: Polysorbate 20) (Uniqema) ⁵ N,N-Dimethyl-N-kokosalkylammoniumbetain (INCI-Bezeichnung: Aqua (Water), Coco-Betaine) (Cognis Deutschland) ⁶ 2-Hydroxyfettalkoholalkoxylat (INCI-Bezeichnung: PPG-1-PEG-9-Lauryl Glykol Ether) (Cognis Deutschland) ⁷ Dimethyldiallylammonium-Acrylsäure Copolymer (INCI-Bezeichnung: Polyquaternium 22) (Nalco) ⁸ "Aqua di Flora" (Dragoco) ⁹ Polyoxethylenoleylether-5-EO-phosphat (INCI-Bezeichnung: Oleth-5-Phosphate) (Croda) ¹⁰ 1-Hydroxyethan-1,1-diphosphonsäure (INCI-Bezeichnung: Etidronic Acid, Aqua (Water)) (Solutia) ¹¹ modifiziertes Kollagenhydrolysat (INCI-Bezeichnung: Potassium Cocoyl Hydrolyzed Collagen) (Cognis Corp., Emery) ¹² kationisches Proteinhydrolysat (INCI-Bezeichnung: Aqua (Water), Laurdimonium Hydroxypropyl Hydrolyzed Wheat Protein, Ethylparaben, Methylparaben) (Cognis Deutschland) ¹³ Poly(dimethyldiallylammoniumchlorid) (INCI-Bezeichnung: Polyquaternium-6) (Nalco) | | | |

Die klaren Wellmittel gemäß Tabelle 3 wurden jeweils bei 0 - +40 °C für eine Dauer von 90 Tagen gelagert. Nach der Lagerung wurde visuell überprüft, ob eine Phasentrennug bzw. eine Trübung der entsprechenden Wellmittel erfolgte.

### Ergebnisse des Vergleichs

A) Die nicht erfindungsgemäßen merkaptanhaltigen Wellmittel 2.6, 2.7 und 2.8 gemäß Tabelle 3 blieben nach dem Lagertest klar, obwohl sie keinen erfindungsgemäßen alkoxylierten Phosphatester enthielten. Das Parfumöl läßt sich demnach in merkaptanhaltige Wellmittel dauerhaft mit Hilfe der herkömmlichen Solubilisatoren einarbeiten.
B) Die nicht erfindungsgemäßen sulfithaltigen Wellmitteln 2.3, 2.4 und 2.5 gemäß Tabelle 2 trübten sich bereits unmittelbar nach der Herstellung ein. Die Trübung ließ sich durch bloßes Rühren nicht entfernen. Das Parfumöl kann somit nachweislich nicht dauerhaft in sulfithaltige Wellmittel mit Hilfe der herkömmlichen Solubilisatoren eingearbeitet werden.
   Im Gegensatz dazu enthielten die zu 2.3, 2.4 und 2.5 analog formulierten erfindungsgemäßen Wellmittel 1.3, 1.4 und 1.5 gemäß Tabelle 1 zusätzlich 1 Gew.% des erfindungsgemäßen alkoxylierten Phosphatesters und wiesen daher nach der Zubereitung und nach dem Lagertest keine Trübung bzw. Phasentrennung auf.
C) Das nicht erfindungsgemäße sulfithaltige Wellmittel 2.1 enthielt 2.0 Gew.% Polyoxethylenoleylether-5-EO-phosphat als nicht erfindungsgemäßen alkoxylierten Phosphatester. Das Wellmittel 2.1 trübte nach der Lagerung ein, während die erfindungsgemäße Rezeptur 1.1 gemäß Tabelle 1 bei identischen Lagerungsbedingungen klar und ohne Phasentrennung blieb. Die erfindungsgemäße Rezeptur 1.1 gemäß Tabelle 1 enthielt 2.0 Gew.% Polyoxethylenoleylether-10-EO-phosphat als erfindungsgemäßen alkoxylierten Phosphatester.
   Selbst bei einer Erhöhung der Menge des nicht erfindungsgemäßen alkoxylierten Phosphatesters auf 5.0 Gew.% (siehe Wellmittel 2.2 gemäß Tabelle 2), trübte sich nachwievor das nicht erfindungsgemäße Wellmittel bereits nach der Herstellung ein.

Durch die Ergebnisse A), B) und C) wird exemplarisch belegt, daß
1. das Solubilisierungsproblem des Parfumöls nur bei sulfithaltigen Wellmitteln auftritt und
2. sich zur Lösung des Problems nur die erfindungsgemäßen alkoxylierten Phosphatester eignen.

## Patentansprüche

1. Keratinreduzierendes Wellmittel zur dauerhaften Verformung keratinhaltiger Fasern, inbesondere menschlicher Haare, **dadurch gekennzeichnet, daß** es
(a) bezogen auf das gesamte Mittel 5-15 Gew.-% keratinreduzierende Verbindung als Hydrogensulfit und/oder Sulfit und/oder Disulfit,
(b) mindestens ein bei 20°C in reinem Wasser unlösliches Parfumöl und
(c) mindestens einen alkoxylierten Phosphatester der Formel I, 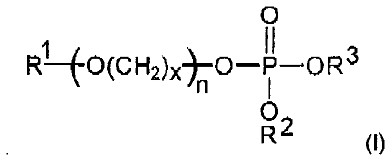 worin
- R¹ steht für eine Arylgruppe, eine lineare oder verzweigte, gesättigte oder ungesättigte C₄-C₃₀-Fettkette,
- R² steht für ein Wasserstoffatom, ein Alkalimetall oder 0.5 Erdalkalimetall,
- R³ steht für ein Wasserstoffatom, eine lineare oder verzweigte, gesättigte oder ungesättigte C₄-C₃₀-Fettkette, ein Alkalimetall, 0.5 Erdalkalimetall, eine Ethylenoxid-Kette mit 6 bis 50 Ethylenoxideinheiten, eine Propylenoxid-Kette mit 6 bis 50 Propylenoxideinheiten,
- n steht für eine ganze Zahl von 6 bis 50
- x steht für eine Zahl 2 oder 3,
enthält.

2. Mittel nach Anspruch 1, **dadurch gekennzeichnet, daß** die keratinreduzierende Verbindung ausgewählt ist aus Natriumdisulfit, Kaliumdisulfit, Ammoniumdisulfit, N-(2-Hydroxyethyl)-ammoniumdisulfit, Ammoniumsulfit, N-(2-Hydroxyethyl)-ammoniumsulfit, Natriumsulfit, Kaliumsulfit Ammoniumhydrogensulfit, N-(2-Hydroxyethyl)-ammoniumhydrogensulfit, Natriumhydrogensulfit und Kaliumhydrogensulfit.

3. Mittel nach einem der Ansprüche 1 oder 2, **dadurch gekennzeichnet, daß** es zusätzlich ein Alkanolamin enthält.

4. Mittel nach Anspruch 3, **dadurch gekennzeichnet, daß** das Alkanolamin ausgewählt ist aus 2-Aminoethanol (Monoethanolamin) und N,N,N-Tris(2-hydroxyethyl)amin (Triethanolamin).

5. Mittel nach einem der Ansprüche 3 oder 4, **dadurch gekennzeichnet, daß** das Alkanolamin in einer Menge von 1-6 Gew.%, bezogen auf das gesamte Mittel, enthalten ist.

6. Mittel nach einem der Ansprüche 1 bis 5, **dadurch gekennzeichnet, daß** der alkoxylierte Phosphatester ausgewählt wird aus Polyoxyethylenoleylether-10-EO-phosphat (INCI: Oleth-10-phosphate) und Polyoxyethyfenoleylether-20-EO-phosphat (INCI: Oleth-20-phosphate).

7. Mittel nach einem der Ansprüche 1 bis 6, **dadurch gekennzeichnet, daß** es zusätzlich als Cosolubilisator mindestens einen weiteren oberflächenaktiven Stoff, der verschieden ist von den alkoxylierten Phosphatestern gemäß Anspruch 1, aus der Gruppe der anionischen, amphoteren, zwitterionischen und nichtionischen Tenside enthält.

8. Mittel nach einem der Ansprüche 1 bis 7, **dadurch gekennzeichnet, daß** es zusätzlich mindestens ein Quellmittel für keratinhaltige Fasern enthält.

9. Mittel nach Anspruch 8, **dadurch gekennzeichnet, daß** das Quellmittel ausgewähit ist aus Glycerin, 1,2-Propylenglykol, Sorbit, Harnstoff und seinen Derivaten, Allantoin, Guanidin, Imidazol und dessen Derivaten.

10. Wellmittel nach einem der Ansprüche 1 bis 9, **dadurch gekennzeichnet, daß** es einen pH-Wert von pH 5 bis 8 besitzt.

11. Verwendung von alkoxylierten Phosphatestern der Formel I gemäß Anspruch 1 zur Solubilisierung von unlöslichen Parfumölen in Wellmitteln gemäß Anspruch 1.

12. Verfahren zur dauerhaften Verformung und gleichzeitigen Parfümierung von keratinhaltigen Fasern, insbesondere menschlichen Haaren, in dem
(i) das gegebenenfalls vorgefeuchtete Haar gegebenenfalls auf Wickelhllfen gewickelt wird,
(ii)ein Wellmittel nach einem der Ansprüch 1 bis 9 auf das Haar aufgetragen und
(iii)nach einer Einwirkzeit vom Haar gespült wird,
(iv) das Haar gegebenenfalls mit einer oxidationsmittelhaltigen Lösung oxidativ fixiert und nach dem Entfernen der Wickelhilfen erneut gespült wird,
(v) das Haar gegebenenfalls einer Nachbehandlung mit einem Nachbehandlungsmittel unterzogen und gegebenenfalls wieder gespült wird.

13. Verfahren nach Anspruch 12, **dadurch gekennzeichnet, daß** das Wellmittel bei Temperaturen von 0-60 °C über eine Einwirkzeit von 5-120 Minuten am Haar belassen wird.

## Claims

1. A keratin-reducing waving agent for the permanent shaping of keratin-containing fibres, in particular human hair, **characterised in that** it contains
(a) relative to the total agent, 5 to 15 wt.% of keratin-reducing compound as hydrogen sulfite and/or sulfite and/or disulfite,
(b) at least one perfume oil that is insoluble in pure water at 20°C and
(c) at least one alkoxylated phosphate ester of formula I, 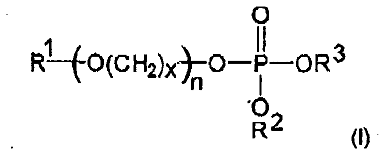 in which
- R¹ denotes an aryl group, a linear or branched, saturated or unsaturated C₄-C₃₀ fatty chain,
- R² denotes a hydrogen atom, an alkali metal or 0.5 alkaline-earth metal,
- R³ denotes a hydrogen atom, a linear or branched, saturated or unsaturated C₄-C₃₀ fatty chain, an alkali metal, 0.5 alkaline-earth metal, an ethylene oxide chain having 6 to 50 ethylene oxide units, a propylene oxide chain having 6 to 50 propylene oxide units,
- n denotes an integer from 6 to 50,
- x denotes a number 2 or 3.

2. The agent according to claim 1, **characterised in that** the keratin-reducing compound is selected from sodium disulfite, potassium disulfite, ammonium disulfite, N-(2-hydroxyethyl)ammonium disulfite, ammonium sulfite, N-(2-hydroxyethyl)ammonium sulfite, sodium sulfite, potassium sulfite, ammonium hydrogen sulfite, N-(2-hydroxyethyl)ammonium hydrogen sulfite, sodium hydrogen sulfite and potassium hydrogen sulfite.

3. The agent according to one of claims 1 or 2, **characterised in that** it additionally contains an alkanol amine.

4. The agent according to claim 3, **characterised in that** the alkanol amine is selected from 2-aminoethanol (monoethanolamine) and N,N,N-tris(2-hydroxyethyl)amine (triethanolamine).

5. The agent according to one of claims 3 or 4, **characterised in that** the alkanol amine is included in an amount from 1 to 6 wt.%, relative to the total agent.

6. The agent according to one of claims 1 to 5, **characterised in that** the alkoxylated phosphate ester is selected from polyoxyethylene oleyl ether 10 EO phosphate (INCI: Oleth-10-phosphate) and polyoxyethylene oleyl ether 20 EO phosphate (INCI: Oleth-20-phosphate).

7. The agent according to one of claims 1 to 6, **characterised in that** it additionally contains as a cosolubiliser at least one further surface-active substance that is different from the alkoxylated phosphate esters according to claim 1, from the group of anionic, amphoteric, zwitterionic and non-ionic surfactants.

8. The agent according to one of claims 1 to 7, **characterised in that** it additionally contains at least one swelling agent for keratin-containing fibres.

9. The agent according to claim 8, **characterised in that** the swelling agent is selected from glycerol, 1,2-propylene glycol, sorbitol, urea and derivatives thereof, allantoin, guanidine, imidazole and derivatives thereof.

10. The waving agent according to one of claims 1 to 9, **characterised in that** it has a pH value of pH 5 to 8.

11. Use of alkoxylated phosphate esters of formula I according to claim 1 to solubilise insoluble perfume oils in waving agents according to claim 1.

12. A method for permanently shaping and simultaneously perfuming keratin-containing fibres, in particular human hair, in which
(i) the optionally pre-wetted hair is optionally wound onto rolling aids,
(ii) a waving agent according to one of claims 1 to 9 is applied to the hair and
(iii) is rinsed from the hair after a contact time,
(iv) the hair is optionally oxidatively fixed with a solution containing an oxidising agent and is rinsed again following removal of the winding aids,
(v) the hair optionally undergoes an aftertreatment with an aftertreatment agent and is optionally rinsed again.

13. The method according to claim 12, **characterised in that** the waving agent is left on the hair at temperatures from 0 to 60°C for a contact time from 5 to 120 minutes.

## Revendications

1. Agent de mise en plis, réduisant la kératine, destiné à la déformation durable de fibres kératiniques, en particulier les cheveux humains, **caractérisé en ce qu'**il contient
(a) par rapport à la totalité de l'agent, 5-15% en poids de composé réduisant la kératine tel que de l'hydrogénosulfite et/ou du sulfite et/ou du disulfite,
(b) au moins une huile parfumée insoluble dans l'eau pure à 20°C et
(c) au moins un ester de phosphate alcoxylé de formule I, 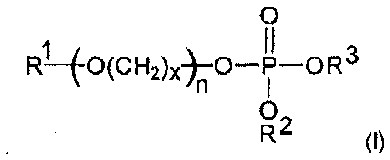 où
- R¹ représente un groupe aryle, une chaîne grasse en C₄-C₃₀ linéaire ou ramifiée, saturée ou insaturée,
- R² représente un atome d'hydrogène, un métal alcalin ou 0,5 métal alcalino-terreux,
- R³ représente un atome d'hydrogène, une chaîne grasse en C₄-C₃₀ linéaire ou ramifiée, saturée ou insaturée, un métal alcalin, 0,5 métal alcalino-terreux, une chaîne d'oxyde d'éthylène comprenant 6 à 50 unités d'oxyde d'éthylène, une chaîne d'oxyde de propylène comprenant 6 à 50 unités d'oxyde de propylène,
- n représente un nombre entier de 6 à 50,
- x représente un nombre 2 ou 3.

2. Agent selon la revendication 1, **caractérisé en ce que** le composé réduisant la kératine est choisi parmi le disulfite de sodium, le disulfite de potassium, le disulfite d'ammonium, le disulfite de N-(2-hydroxyéthyl)-ammonium, le sulfite d'ammonium, le sulfite de N-(2-hydroxyéthyl)-ammonium, le sulfite de sodium, le sulfite de potassium, l'hydrogénosulfite d'ammonium, l'hydrogénosulfite de N-(2-hydroxyéthyl)-ammonium, l'hydrogénosulfite de sodium et l'hydrogénosulfite de potassium.

3. Agent selon l'une quelconque des revendications 1 ou 2, **caractérisé en ce qu'**il contient en plus une alcanolamine.

4. Agent selon la revendication 3, **caractérisé en ce que** l'alcanolamine est choisie parmi le 2-aminoéthanol (monoéthanolamine) et la N,N,N-tris(2-hydroxyéthyl)amine (triéthanolamine).

5. Agent selon l'une quelconque des revendications 3 ou 4, **caractérisé en ce que** l'alcanolamine est contenue en une quantité de 1-6% en poids, par rapport à la totalité de l'agent.

6. Agent selon l'une quelconque des revendications 1 à 5, **caractérisé en ce que** l'ester de phosphate alcoxylé est choisi parmi le polyoxyéthylèneoléyléther-10-OE-phosphate (INCI : Oleth-10-phosphate) et le polyoxyéthylèneoléyléther-20-OE-phosphate (INCI: Oleth-20-phosphate).

7. Agent selon l'une quelconque des revendications 1 à 6, **caractérisé en ce qu'**il contient en outre, comme cosolubilisant, au moins une autre substance tensioactive, qui est différente des esters de phosphate alcoxylés selon la revendication 1, du groupe des agents tensioactifs anioniques, amphotères, zwittérioniques et non ioniques.

8. Agent selon l'une quelconque des revendications 1 à 7, **caractérisé en ce qu'**il contient en outre au moins un agent gonflant pour des fibres kératiniques.

9. Agent selon la revendication 8, **caractérisé en ce que** l'agent gonflant est choisi parmi le glycérol, le 1,2-propylèneglycol, le sorbitol, l'urée et ses dérivés, l'allantoïne, la guanidine, l'imidazole et ses dérivés.

10. Agent de mise en plis selon l'une quelconque des revendications 1 à 9, **caractérisé en ce qu'**il présente un pH de 5 à 8.

11. Utilisation d'esters de phosphate alcoxylés de formule I selon la revendication 1 pour la solubilisation d'huiles parfumées insolubles dans les agents de mise en plis selon la revendication 1.

12. Procédé pour la mise en forme durable et pour parfumer simultanément des fibres kératiniques, en particulier des cheveux humains, dans lequel
(i) on enroule les cheveux le cas échéant humidifiés au préalable sur des supports d'enroulement,
(ii) un agent de mise en plis selon l'une quelconque des revendications 1 à 9 est appliqué sur les cheveux et
(iii) éliminé par rinçage après un temps d'action sur les cheveux,
(iv) les cheveux sont le cas échéant fixés par oxydation à l'aide d'une solution contenant un oxydant et à nouveau rincés après l'élimination des supports d'enroulement,
(v) les cheveux sont le cas échéant soumis à un post-traitement à l'aide d'un agent de post-traitement et le cas échéant à nouveau rincés.

13. Procédé selon la revendication 12, **caractérisé en ce que** l'agent de mise en plis est laissé sur les cheveux à des températures de 0-60°C pendant un temps d'action de 5-120 minutes.
